# EUROPEAN PATENT SPECIFICATION

(11) **EP 0 586 960 B1**
(45) Date of publication and mention of the grant of the patent: **07.02.1996**
(21) Application number: 93113466.2
(22) Date of filing: 24.08.1993
(51) Int. Cl.: C07D 311/86, A61K 31/35

(54) **Xanthone derivatives, their preparation and use**
Xanthon-Derivate, Verfahren zu ihrer Herstellung und ihrer Verwendung
Dérivés de xanthone procédé pour leur préparation et leur utilisation

(30) Priority: 07.09.1992 IT MI922071
(43) Date of publication of application: 16.03.1994
(73) Proprietor: INVERNI DELLA BEFFA S.P.A., I-20141 Milano (IT)
(72) Inventor: Da Re, Paolo, I-20141 Milano (IT); Pifferi, Giorgio, I-20141 Milano (IT); Valenti, Piero, I-20141 Milano (IT); Malandrino, Salvatore, I-20141 Milano (IT)
(74) Representative: Minoja, Fabrizio

(56) References cited:
- EP-A- 0 093 381
- EP-A- 0 175 376
- PATENT ABSTRACTS OF JAPAN vol. 012, no. 426 (C-542)10 November 1988 & JP-A-63156720
- CHEMICAL ABSTRACTS, vol. 75, 1971, Columbus, Ohio, US; abstract no. 5706t, 'Antiinflammatory, antisecretory and antiulcerogenic tertiary-aminoalkoxy-9- benzylxanthenes and thioxanthenes' page 474-475 ;& US-A-3555043
- CHEMICAL ABSTRACTS, vol. 60, no. 13, 1964, Columbus, Ohio, US; abstract no. 15847f, 'Furano compounds'& JOURNAL OF ORGANIC CHEMISTRY vol. 29, no.5, 1964, page 1089-92
- CHEMICAL ABSTRACTS, vol. 82, 1975, Columbus, Ohio, US; abstract no. 170596j, 'Reactions of 3,6-diethoxyxanthen-9-one with alkylaluminium compounds' page 508
- CHEMICAL ABSTRACTS, vol. 86, 1977, Columbus, Ohio, US; abstract no. 155457d, 'A new synthesis of 9-xanthenones by the reaction of 2-hydroxybenzophenones with metal salts' page 453 ;

## Description

The present invention refers to new xanthone derivatives, to their synthesis and to their pharmacological use. More particularly, the invention refers to xanthen-9-one derivatives disubstituted at the positions 3 and 6, having the general formula 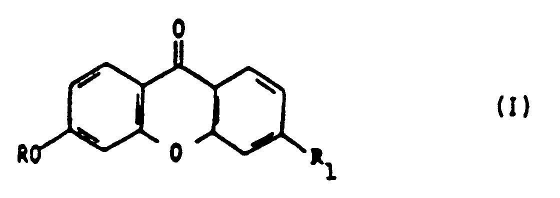
wherein R - C₃-C₈ alkyl (Linear, branched or cyclic) and
R₁ = C₁-C₈ alkyl (linear, branched or cyclic) or an OR group wherein R is as above defined.

In the scientific literature, some synthetic xanthones having a remarkable interest in the pharmaceutical field, are reported.

Between the most known compounds, reference is made to Mepixanox (P. Da Re et al., J. Med. Chem., 13, 527, 1970) active on the central nervous system as breath stimulant, xanthon-4-acetic acid having antitumor activity (G.W. Rawcastle et al., J. Med. Chem., 34, 2864, 1991) and a series of xanthon-2-carboxylic acids (AH7725, Xanoxic acid, Sudexanox) developed as anti-allergic (Drugs of the Future, 1, 313, 1976; ibidem, 1, 43, 1976; ibidem, 4, 736, 1979). Some xanthone derivatives of natural origin show different pharmacological properties, such as inhibition of mono-aminooxidase, antiinflammatory activity and antimicrobial activity (K. Hostettmann et al., Methods Plant Bioch., 1, 493, 1989).

It has now been surprisingly found that xanthones of formula I are endowed with bone resorption inhibiting activity so that their use as anti-osteoporosis agents can be envisaged.

The xanthones of the invention wherein R₁ is alkoxy have been prepared by alkylation of 3,6-dihydroxyxanthone II with suitable alkylating agents, according to the scheme: 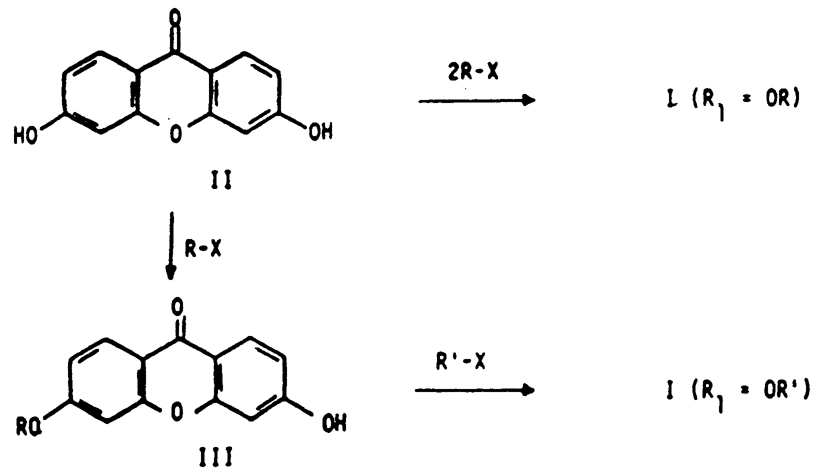
The starting compound, 3,6-dihydroxyxanthone II may be prepared according to the method of P.K. Grover et al. (J. Chem. Soc. 3982, 1955).

The 3,6-dihydroxyxanthone II is O-alkylated according to conventional etherification methods using as alkylating agent an alkyl halide (Example 1a and 1b) or an alkyl sulphate (Example 1c) in a polar solvent, such as, for instance, N,N-dimethylformamide, dimethylsulfoxide, acetone, or methylethylketone performing the reaction in the presence of a base and at variable temperatures depending on the reactivity of the alkylating agent. The alkylated product is recovered from the reaction mixture and purified by crystallisation from a suitable solvent.

The alkylation reaction may be accelerated when carried out in the presence of a phase-transfer catalyst. For instance the synthesis of I (R = iPr, R₁ = OiPr) was carried out also in a biphasic system consisting of toluene and water, in the presence of an excess isopropylbromide and of sodium hydroxide and by catalysis with a quaternary ammonium salt, such as N-benzyltriethylammonium chloride.

The mixture is refluxed under stirring for 5 hours, cooled to room temperature and the reaction product is recovered from the organic phase by evaporating under vacuum and subsequent crystallization (Example 1a). Using only one mole of R-X halide it is possible to recover from the reaction mixture the monoether (Example 2a) which may then be further alkylated to give the 3,6-dialkoxy compounds I, symmetric and, with a different alkylating agent (R'-X), asymmetric ones (Example 2b).

The preparation of the compounds of formula I wherein R₁ is C₁-C₈ alkyl is carried out according to the following scheme 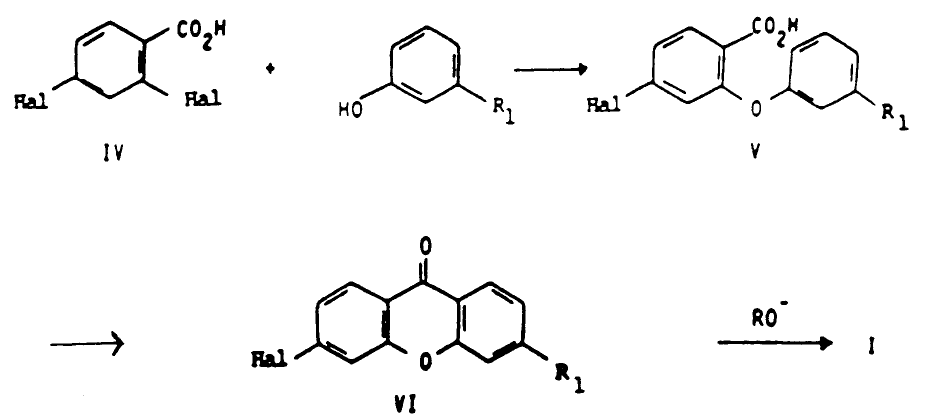
by nucleophilic substitution on the 2,4-dihalobenzoic acids (IV, Hal = F, Cl, Br, J) with 3-alkylphenols (wherein R₁ is C₁-C₈ alkyl) in the presence of powder copper, potassium carbonate and copper (I) iodide in high-boiling aprotic solvent. The diphenylethers V (Examples 3a and 4a) are obtained, which are cyclized in the presence of a dehydrating agent at high temperature.

The so obtained 3-halogen-6-alkylxanthones VI (Examples 3b and 4b) are finally subjected to a second nucleophilic substitution with alkoxides RO⁻, to give the compounds I wherein R₁ is an alkyl group, as above defined (Examples 3c, 3d and 4c). The reaction is preferably carried out by heating the compounds VI with a sodium or potassium alcoholate in alcoholic medium or alcohol-dioxane, at temperatures ranging from 70 to 150°C, optionally in the presence of phase-transfer catalysts.

The activity of the new xanthones has been evaluated in vitro and in vivo. The effect on the bone resorption has been studied in vitro according to Zambonin-Zallone et al. Anatomy Embriology, 165, 405, 1982). The medullary bone, deriving from tibia and femur of hens fed with hypocalcic diet for 7 days was washed with MEM buffer, modified according to Joklik, at 4°C and filtered through a nylon filter with pore diameters of 112 microns. The obtained cell suspension was centrifuged at 1800 rpm for 5 minutes. The surnatant was discarded and the pellet treated with a 0.2% NaCl solution for 30 seconds to eliminate most of the present red blood cells. After restoration of the physiological osmolarity by means of 1.6% NaCl, the sample was centrifuged at 1800 rpm for 5 min. The obtained pellet, suspended again in the culture medium, was layered on 75% foetal calf serum in Joklik MEM for 45 min, so as to obtain a cell suspension enriched in osteoclasts. The fibroblasts were then filtered off by means of two filtrations on 112 µn nylon filters.

The so obtained cells were cultivated in a nucleotide-free medium, in the presence of 3 µg/ml of cytosine-1-D-β-arabinofuranoside to block the mitosis of proliferating cells. The culture medium contained 100 µg/ml streptomycin, 100 U.I./ml penicillin and 10% fetal calf serum. The cultures were incubated in humid atmosphere saturated with 5% CO₂ at 37°C and washed after 24 and 48 hours to remove non-adhering medullary cells.

The bone resorption was evaluated using the so prepared osteoclasts cultures cultured in the presence of rat bone particles, pre-labelled in vivo with trititated proline. During the resorption process, the collagen was degraded into fragments containing tritiated proline which was released in the culture medium; the resorption was calculated by measuring the radioactivity present in the medium after 24 and 48 hours of treatment.

Simultaneously to each experiment, cell-free samples have also been prepared containing only bone fragments labelled in the culture medium to measure the aspecific release of radioactivity from the bone. This value was subtracted as background from the corresponding experimental value.

The compounds under examination were added to the culture medium at the concentrations of 10 and 25 µg/ml dissolved in DMSO. The inhibiting effect on the bone resorption of osteoclast of compound I (R = iPr, R₁ = OiPr), as an example, is reported on Table 1.

**Table 1**

| Effect of the compound I (R = R' = OiPr) on bone resorption of hen osteoclasts | | | | | |
|---|---|---|---|---|---|
| Compound | Conc. µg/ml | Bone Res. µg | | Inhibition % vs. controls | |
| | | 24h | 48h | 24h | 48h |
| Controls (DMSO) | - | 44.3±7.5 | 72.7±9.6 | - | - |
| I (R=iPr; | 10 | 32.1±3.6 | 50.2±6.3 | 27.5 | 30.9 |
| R₁=OiPr) | 25 | 16.0±1.0* | 21.0±1.0* | 63.8 | 71.1 |
| n = 4 | | | | | |

| | | | | | |
|---|---|---|---|---|---|
| *p < 0.01 | | | | | |

At the concentrations of 10 and 25 µg/ml, the compound showed a dose dependent activity. Particularly, the bone resorption inhibition was higher than 60% in comparison to unexposed controls, both at 24 and at 48 hours.

The significance was evaluated by the Student's t test versus the group treated with the solvent alone (control).

In vivo, the antiosteoporotic activity of the compound I (R = iPr, R₁ = OiPr) administered by the oral route, was evaluated on suckling rats under hypocalcic diet according to the method, slightly modified, disclosed by Lozupone et al., Bone, 9; 215, 1988). Female Wistar rats were allowed to delivery spontaneously.

The offsprings were pooled, weighed and re-distributed so as to obtain nests similar in weight and number.

The mothers during the suckling period were fed with an hypocalcic diet (Altromin DP 1031) and divided in two groups.

One group was treated orally with compound I (R = iPr, R₁ = OiPr) suspended in Methocel 0.5% at the dose of 250 mg/kg, whereas the second was treated with the vehicle alone (control group). The animals were sacrificed after 10 days from the start of milking.

The femur was taken from each animal and fixed, included in methacrylate and transversally cut. Two sections taken at the distal methaphysis and one from the medial methaphysis were microradiographed so as to measure the thickness of the compacta.

The results are reported in Table 2.

**Table 2**

| Compound | Dose mg/kg/os | Compacta thinkness | | | |
|---|---|---|---|---|---|
| | | Diaphysis | | Methaphysis | |
| | | mm ± S.D. | % | mm ± S.D. | % |
| Control (Methocel 0.5%) | - | 0.55±0.04 | - | 0.31±0.08 | - |
| I (R=iPr; | | | | | |
| R₁=OiPr) | 250 | 0.71±0.03* | +29 | 0.40±0.12* | +29 |
| n = 3 | | | | | |

| | | | | | |
|---|---|---|---|---|---|
| *p < 0.05 | | | | | |

The compound under examination shows a significant antiosteoporotic activity, the thickness being 29% higher than in the control group both in the diaphysis and in the methaphysis.

The acute toxicity of the compound I (R = iPr; R₁ = OiPr) was evaluated in the rat after oral and intraperitoneal administration.

Sprague-Dawley rats of both sexes (5M + 5F) were used for each group. After treatment, the animals were observed for 14 days and the LD₅₀ was calculated with the probit methods (Finney D.J. in "Probit Analysis", Cambridge University Press, 3ª Ed., Cambridge, 1971). The compound has an LD₅₀ > 4000 mg/kg (LD₀) after oral administration and an LD₅₀ > 1000 mg/kg (LD₂₀) by intraperitoneal route.

The significant bone resorption inhibiting activity and the low acute toxicity make the compounds of the invention particularly suitable as anti-osteoporotic agents in man.

### Example 1

a) 3,6-Di(isopropoxy)xanthone (I, R = iPr, R₁ = OiPr)
   A mixture of 27.5 g (0.121 mol) of 3,6-dihydroxyxanthone (II), 54.6 ml (0.546 mol) of isopropylbromide and 27.5 g of N-benzyltriethylammonium chloride in 1 l toluene and 580 ml of 50% aqueous sodium hydroxide was refluxed under stirring for 5 hours.
   The mixture was cooled to room temperature and the organic phase was separated, washed with water to neutrality, dried on sodium sulphate and evaporated.
   The residue was crystallized from ligroine to yield 31 g (84%) of I (R = iPr, R₁ = OiPr) m.p. 135°C (M⁺ at m/z 312).
b) 3,6-Di(cyclopentyloxy)xanthone (I, R = C₅H₉, R₁ = OC₅H₉)
   1.2 g (0.005 mol) of dihydroxyxanthone (II), 2 g of anhydrous potassium carbonate and 2 ml (0.019 mol) of cyclopentylbromide in 60 ml of dimethylformamide were refluxed under stirring for 14 hours.
   The dimethylformamide was evaporated and the residue was dissolved in water and methylene chloride, which was separated, washed with water, dried on sodium sulphate and evaporated. The residue was crystallized from ligroine to give 1.2 g (63%) of I (R = C₅H₉, R₁ = OC₅H₉), m.p. 143-145°C (M⁺ at m/z 364).
c) 3,6-Di(n-butoxy)xanthone (I, R = n-Bu, R₁ = n-BuO 500 mg of anhydrous potassium carbonate and 550 mg (2.6 mmol) of di-n-butylsulfate were added to a solution of 300 mg (1.3 mmol) of dihydroxyxanthone (II) in 20 ml of dimethylformamide and the mixture was refluxed under stirring for 7 hours.
   The mixture was poured into water, filtered, washed and dried. 200 mg (45%) of I (R = n-Bu, R₁ = n-BuO) were obtained after crystallization from ligroine, m.p. 84-86°C (M⁺ at m/z 340).

### Example 2

a) 3-Isopropoxy-6-hydroxyxanthone (III, R = iPr)
   1 g (0.005 mol) of dihydroxyxanthone (II) were suspended in 50 ml of toluene, 1 g of N-benzyltriethylammonium chloride and 0.5 ml (0.005 mmol) of isopropyl bromide were added and then 25 ml of 50% NaOH. The mixture was refluxed under stirring for 6 hours and the phases were separated: the toluene phase gave after evaporation 0.28 g of 3,6-diisopropoxyxanthone. The aqueous phase was acidified and the solid was filtered and dried. Recrystallization from toluene afforded 0.26 g of product (III, R = iPr), m.p. 268-281°C (M⁺ at m/z 270).
b) 3-Isopropoxy-6-n-propoxy-xanthone (I, R = iPr, R₁ = OPr)
   0.16 g of the previous compound (III, R = iPr), 0.2 g of anhydrous K₂CO₃ and 0.1 ml of iodopropane in 20 ml of acetone were refluxed under stirring for 9 hours.
   The mixture was filtered at high temperature, washed with acetone and evaporated. The residue crystallized from ligroine gave 100 mg of product having m.p. 78-80°C (M⁺ at m/z 312).

### Example 3

a) 2-(3-Methylphenoxy)-4-fluorobenzoic acid (V, Hal = F, R₁ = Me)
   A mixture of 3.8 g (0.014 mol) of 2-iodo-4-fluorobenzoic acid (IV, Hal(2) = J, Hal(4) = F), 1.5 ml (0.014 mol) of m-cresol, 3.5 g of anhydrous K₂CO₃, 0.5 g of copper powder and 0.5 g of CuI in 40 ml of nitrobenzene were heated 170-180°C for 8 hours under stirring. The nitrobenzene was steam distilled and the mixture was filtered and acidified with diluted HCl.
   After filtration and washing with water, the residue was dissolved in aqueous bicarbonate and acidified again with diluted HCl. The product was filtered, washed and dried to give 1.8 g (52% yield) of V (Hal = F, R₁ = Me), m.p. 110-112°C (M⁺ at m/z 246).
b) 3-Methyl-6-fluoroxanthone (VI, Hal = F, R₁ = Me) 20 g of phosphoric anhydride and then 1.8 g of the previous compound (V, Hal = F, R₁ = Me) were added to 20 ml of 85% phosphoric acid and the mixture was heated to 120°C under stirring for 5 hours.
   The reaction mixture was then poured into ice, filtered, washed with water and dried: about 2 g of a mixture of 1-methyl-6-fluoroxanthone and of 3-methyl-6-fluoroxanthone were obtained which was chromatographed on silica gel.
   6.8 g of the impurity 1-methyl-6-fluoro-xanthone and 0.7 g of 3-methyl-6-fluoro-xanthone (VI, Hal = F, R₁ = Me), m.p. 161-162°C (M⁺ at m/z 228) were recovered.
c) 3-Methyl-6-cyclopentyloxyxanthone (I, R = C₅H₉, R₁ = Me)
   A solution of 1 g of sodium metal in 30 ml of cyclopentanol was prepared. 1 g of the previous 3-methyl-6-fluoroxanthone (VI, Hal = F, R₁ = Me) was separately dissolved in 30 ml of dioxane. The two solutions were mixed and refluxed for 100 hours. The mixture was evaporated, the residue was dissolved with diluted HCl and stirred for 1 hour. After extraction with methylene chloride, the mixture was washed with diluted HCl and then with water, dried on sodium sulphate and evaporated. The residue was chromatographed on silica gel eluting with petroleum ether:ethyl acetate 9:1 and crystallized from ligroine to give 0.22 g of I (R = C₅H₉, R₁ = Me), m.p. 105-106°C (M⁺ at m/z 294).
d) 3-Methyl-6-isopropoxyxanthone (I, R = iPr, R₁ = Me)
   A solution of 330 mg (1.45 mmol) of 3-methyl-6-fluoroxanthone (VI, Hal = F, R₁ = Me) in 10 ml of isopropanol was added to a solution of 740 mg of KOH in 10 ml of isopropanol. 4.2 g of tetrabutylammonium bromide were then added and the mixture was heated to 75°C for 6 hours under stirring. The isopropanol was evaporated, the residue dissolved with diluted HCl and extracted with CH₂Cl₂. The organic phase was evaporated, washed with diluted HCl, then with water, dried and evaporated. The residue was chromatographed on silica gel eluting with toluene.
   The isolated product crystallized from ligroine gave 100 mg (26%) of I (R = iPr, R₁ = Me), m.p. 94-96°C (M⁺ at m/z 268).

### Example 4

a) 2-(3-Methylphenoxy)-4-chlorobenzoic acid (V, Hal = Cl, R₁ = Me)
   A mixture of 57.3 g (0.3 mol) of 2,4-dichlorobenzoic acid (VI, Hal = Cl, R₁, = Me), 32.4 g (0.3 mol) of m-cresol, 100 g of anhydrous potassium carbonate, 2 g of copper powder and 2 g of CuI in 500 ml of nitrobenzene was refluxed at 170-180°C for 6 hours under stirring. The nitrobenzene was steam distilled, filtered and acidified with diluted HCl. The mixture was filtered, washed, dried, extracted with hot ligroine, from which the product crystallized. 52 g of product V were obtained (66% yield) (R₁ = Me, Hal = Cl) having m.p. 153-155°C (M⁺ at m/z 262).
b) 3-Methyl-6-chloroxanthone (VI, Hal = Cl, R₁ = Me) 500 g of phosphoric anhydride and then 50 g of the previous product (V, Hal = Cl, R₁ = Me) were added to 500 ml of 85% phosphoric acid; the mixture was stirred at 100°C for 3 hours. The mixture was poured into ice, filtered, washed with water and dried: about 40 g of a mixture of 1-methyl-6-chloroxanthone and of 3-methyl-6-chloroxanthone was obtained.
   20 g of the previous mixture were chromatographed on silica gel column. The impurity 1-methyl-6-chloroxanthone (7 g, m.p. 111-113°C) was first isolated and then 7.5 g of 3-methyl-6-chloroxanthone (VI, Hal = Cl, R₁ = Me) having m.p. 144-147°C (M⁺ at m/z 244).
c) 3-Methyl-6-isopropoxyxanthone (I, R = iPr, R₁ = Me)
   A solution of 350 mg (1.43 mmol) of 3-methyl-6-chloroxanthone (VI, Hal = Cl, R₁ = Me) in 10 ml of isopropanol was added to a solution of 740 mg of KOH in 10 ml of isopropanol.
   4.2 g of tetrabutylammonium bromide were then added and the mixture was then stirred at 75°C for 6 hours. The solvent was evaporated, the residue was dissolved in diluted HCl and extracted with CH₂Cl₂. The organic phase was evaporated, washed with diluted HCl, then with water, dried and evaporated. The residue was chromatographed on silica gel eluting with toluene. The isolated product was crystallized from ligroine to give 80 mg (21%) of I (R = iPr, R₁ = Me), m.p. 94-96°C (M⁺ at m/z 268).

## Claims

1. 3,6-Disubstituted xanthen-9-one derivatives of formula I 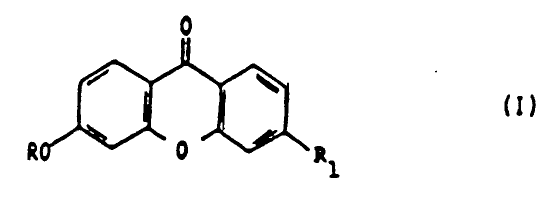 wherein R = C₃-C₈ alkyl (linear, branched or cyclic) and
R₁ = C₁-C₈ alkyl (linear, branched or cyclic) or an OR group wherein R is as above defined.

2. A compound according to claim 1, selected from 3,6-di(isopropoxy)xanthone, 3,6-di(n-butoxy)xanthone, 3,6-di(cyclopentyloxy)xanthone, 3-isopropoxy-6-n-propoxy-xanthone, 3-methyl-6-isopropoxy-xanthone and 3-methyl-6-cyclopentyloxy-xanthone.

3. A process for the preparation of the compounds of formula I according to claim 1 wherein R₁ = OR, characterized in that 3,6-dihydroxy-xanthone (II) is alkylated with an excess of the same alkylating agent R-X or in subsequent phases with two different alkylating agents R-X and R'-X in the presence of a base, according to the scheme 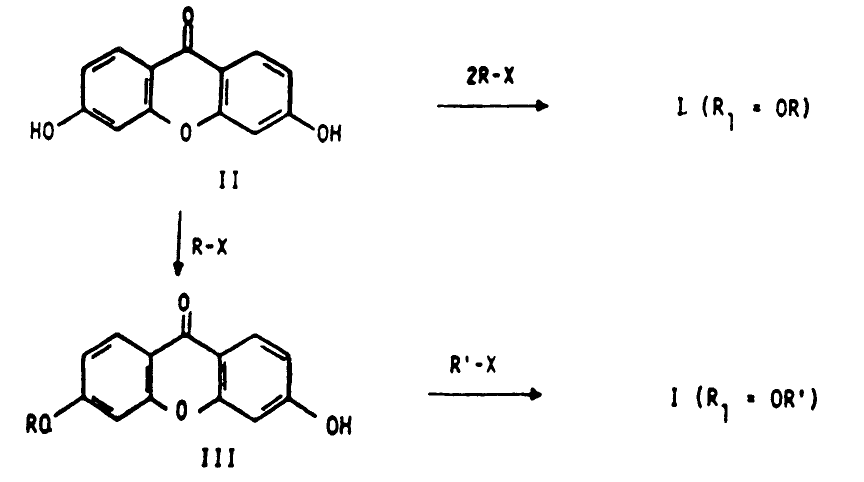 wherein R and R' are C₃-C₈ alkyl (linear, branched or cyclic) wherein X is an halogen atom or a residue ROSO₃⁻ or R'OSO₃⁻.

4. A process according to claim 3, characterized in that the reaction is carried out in polar solvents.

5. A process according to claim 4, wherein the solvent is selected from N,N'-dimethylformamide, dimethylsulfoxide, acetone and methylethylketone.

6. A process according to claim 5 characterized in that the reaction is carried out in the presence of a phase-transfer catalyst, in a biphasic system consisting of water and a water-immiscible solvent.

7. A process for the preparation of the compounds of formula I wherein R₁ = C₁-C₈ alkyl (linear, branched or cyclic) characterized in that 3-halogen-6-alkyl-xanthone of formula VI is reacted with an alkoxide RO⁻, the compound of formula VI being prepared according to the following scheme 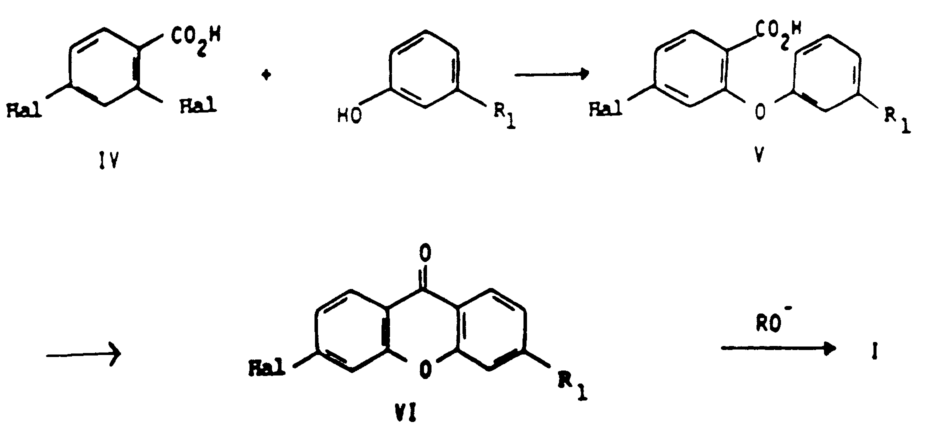 wherein Hal = F, Cl, Br, J and R₁ is a C₁-C₈ alkyl (linear, branched or cyclic) and R is as defined above.

8. A process according to claim 7, wherein the reaction between alkoxide RO⁻ and 3-halogen-6-alkyl-xanthone VI is carried out at temperatures ranging from 70 to 150°C, optionally also in the presence of a phase transfer catalyst.

9. Pharmaceutical compositions containing as the active principle a compound of claims 1-2.

10. Use of the compounds of claims 1-2 for the preparation of a medicament having anti-osteoporotic activity.

## Patentansprüche

1. 3,6-Disubstituierte Xanthen-9-on-Derivate der Formel (I) 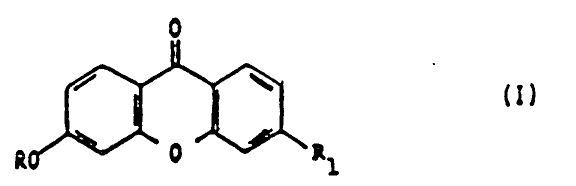 worin
R = C₃-C₈-Alkyl (linear, verzweigt oder cyclisch)
und
R₁ = C₁-C₈-Alkyl (linear, verzweigt oder cyclisch) oder eine Gruppe OR, worin R wie oben definiert ist.

2. Verbindung nach Anspruch 1, ausgewählt aus der Gruppe 3,6-Di-(isopropoxy)xanthon, 3,6-Di-(n-butoxy)xanthon, 3,6-Di-(cyclopentyloxy)xanthon, 3-Isopropoxy-6-n-propoxy-xanthon, 3-Methyl-6-isopropoxy-xanthon und 3-Methyl-6-cyclopentyloxy-xanthon.

3. Verfahren zur Herstellung der Verbindungen der Formel (I) nach Anspruch 1, worin R₁ = OR, dadurch gekennzeichnet, daß 3,6-Dihydroxy-xanthon (II) mit einem Überschuß des gleichen Alkylierungsmittels R-X alkyliert wird oder in nachfolgenden Phasen mit zwei unterschiedlichen Alkylierungsmitteln R-X und R'-X in Gegenwart einer Base alkyliert wird entsprechend dem Reaktionsschema 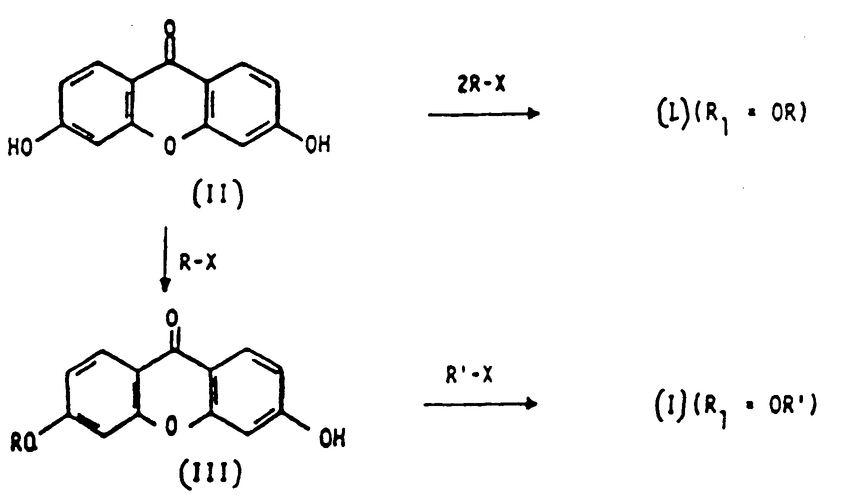 worin R und R' für C₃-C₈-Alkyl (linear, verzweigt oder cyclisch) stehen, worin X ein Halogenatom oder einen Rest ROSO₃⁻ oder R'OSO₃⁻ bedeutet.

4. Verfahren nach Anspruch 3, dadurch gekennzeichnet, daß die Reaktion in polaren Lösungsmitteln durchgeführt wird.

5. Verfahren nach Anspruch 4, worin das Lösungsmittel ausgewählt wird aus der Gruppe N,N'-Dimethylformamid, Dimethylsulfoxid, Aceton und Methylethylketon.

6. Verfahren nach Anspruch 5, dadurch gekennzeichnet, daß die Reaktion in Gegenwart eines Phasenübergangskatalysators in einem biphasischen System, das aus Wasser und einem mit Wasser nicht mischbaren Lösungsmittel besteht, durchgeführt wird.

7. Verfahren zur Herstellung der Verbindungen der Formel (I), worin R₁ = C₁-C₈-Alkyl (linear, verzweigt oder cyclisch), dadurch gekennzeichnet, daß 3-Halogen-6-alkylxanthon der Formel (VI) mit einem Alkoxid RO⁻ umgesetzt wird, wobei die Verbindung der Formel (VI) nach dem folgenden Reaktionsschema hergestellt wird: 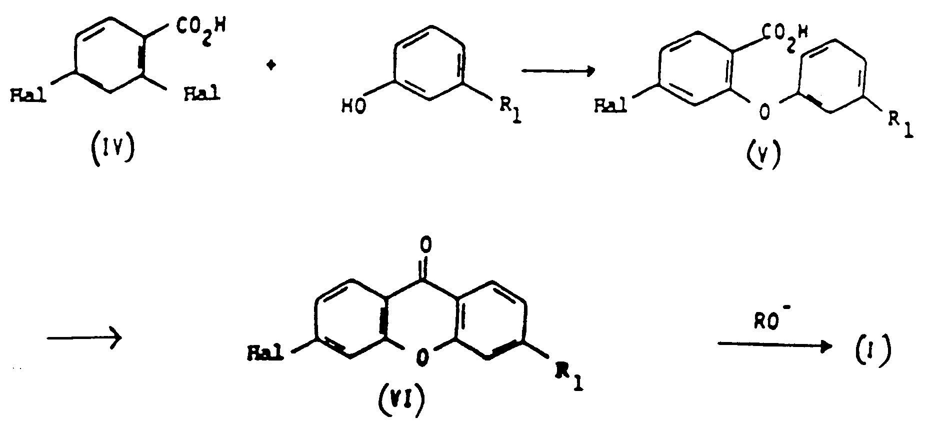 worin Hal = F, Cl, Br, J und R₁ steht für ein C₁-C₈-Alkyl (linear, verzweigt oder cyclisch) und R wie oben definiert ist.

8. Verfahren nach Anspruch 7, dadurch gekennzeichnet, daß die Umsetzung zwischen dem Alkoxid RO⁻ und 3-Halogen-6-alkylxanthon (VI) bei Temperaturen in dem Bereich von 70 bis 150°C, gegebenenfalls auch in Gegenwart eines Phasenübergangskatalysators, durchgeführt wird.

9. Pharmazeutische Zusammensetzungen, die als aktives Prinzip (Wirkstoff) eine Verbindung der Ansprüche 1 bis 2 enthalten.

10. Verwendung der Verbindungen nach den Ansprüchen 1 bis 2 zur Herstellung eines Arzneimittels mit einer Antiosteoporose-Aktivität.

## Revendications

1. Dérivés de xanthen-9-one 3,6-disubstituée, de formule I : 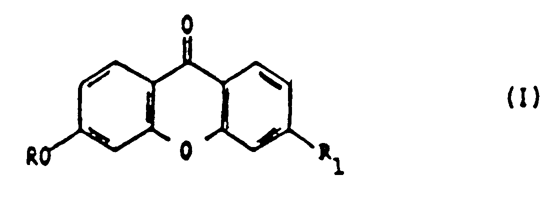 dans laquelle R = alkyle en C₃-C₈ (linéaire, ramifié ou cyclique) et R₁ = alkyle en C₁-C₈ (linéaire, ramifié ou cyclique) ou un groupe OR où R est tel que défini ci-dessus.

2. Composé selon la revendication 1, choisi par la 3,6-di(isopropoxy)xanthone, la 3,6-di(n-butoxy)xanthone, la 3,6-di(cyclopentyloxy)xanthone, la 3-isopropoxy-6-n-propoxy-xanthone, la 3-méthyl-6-isopropoxy-xanthone et la 3-méthyl-6-cyclopentyloxy-xanthone.

3. Procédé pour la préparation des composés de formule I selon la revendication 1 pour lesquels R₁ = OR, caractérisé en ce que la 3,6-dihydroxy-xanthone (II) est alkylée avec un excès du même agent d'alkylation R-X ou, en phases successives, avec deux agents d'alkylation différents R-X et R'-X en présence d'une base, selon le schéma : 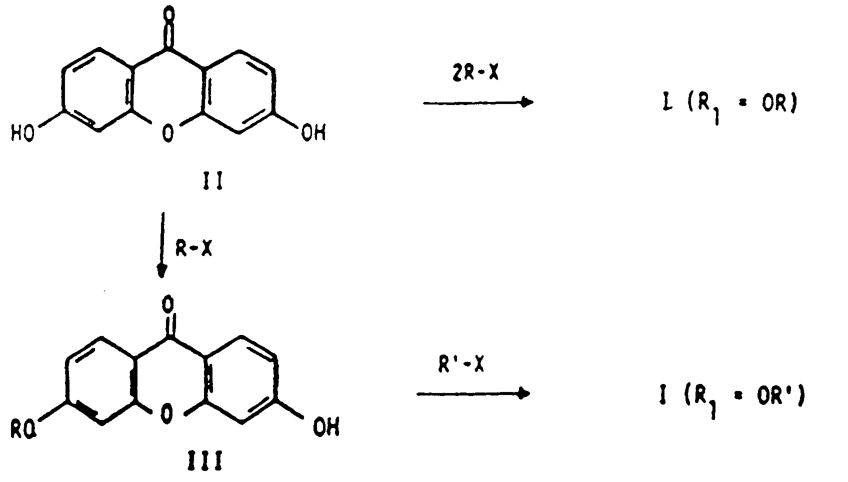 où R et R' représentent alkyle en C₃-C₈ (linéaire, ramifié ou cyclique) et où X est un atome d'halogène ou un reste ROSO₃⁻ ou R'OSO₃⁻.

4. Procédé selon la revendication 3, caractérisé en ce que la réaction est effectuée dans des solvants polaires.

5. Procédé selon la revendication 4, dans lequel le solvant est choisi parmi le N,N'-diméthylformamide, le diméthylsulfoxyde, l'acétone et la méthyléthylcétone.

6. Procédé selon la revendication 5, caractérisé en ce que la réaction est effectuée en présence d'un catalyseur de transfert de phase, dans un système biphasique constitué par de l'eau et d'un solvant non miscible à l'eau.

7. Procédé de préparation des composés de formule I pour lesquels R₁ = alkyle en C₁-C₈ (linéaire, ramifié ou cyclique), caractérisé en ce qu'une 3-halogèno-6-alkylxanthone de formule VI est mise à réagir avec un alcoolate RO⁻, le composé de formule VI étant préparé selon le schéma suivant : 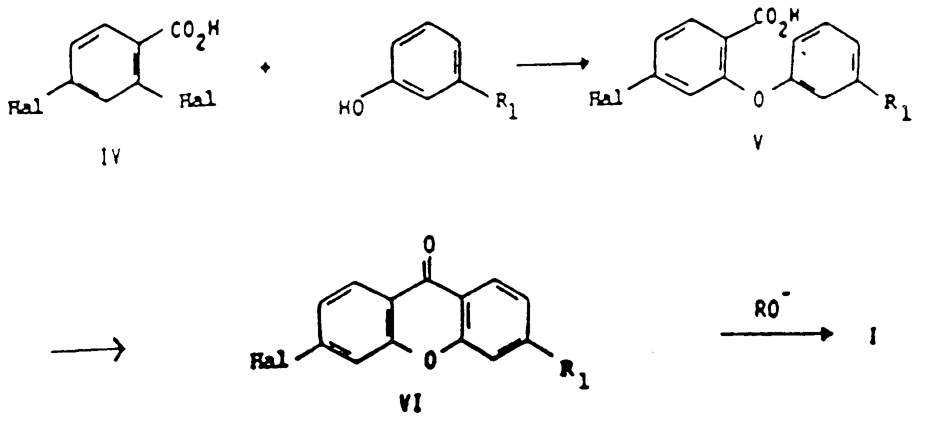 où Hal = F, Cl, Br, I et R₁ est ul alkyle en C₁-C₈ (linéaire, ramifié ou cyclique) et R est tel que défini ci-dessus.

8. Procédé selon la revendication 7, dans lequel la réaction entre l'alcoolate RO⁻ et la 3-halogèno-6-alkylxanthone VI est effectuée à des températures situées dans la plage allant de 70 à 150 °C, éventuellement également en présence d'un catalyseur de transfert de phase.

9. Compositions pharmaceutiques contenant, en tant que principe actif, un composé selon les revendications 1 et 2.

10. Utilisation des composés selon les revendications 1 et 2 pour la préparation d'un médicament ayant une activité anti-ostéoporose.
